# EUROPEAN PATENT APPLICATION

(11) **EP 2 682 106 A1**
(43) Date of publication of application: **08.01.2014**
(21) Application number: 12174825.5
(22) Date of filing: 03.07.2012
(51) Int. Cl.: A61K 9/127, A61K 47/24

(54) **Method of solubilizing biologically active compounds**

(71) Applicant: PHARES PHARMACEUTICAL RESEARCH N.V., Curaçao (AN)
(72) Inventor: Leigh, Steve, 1000CX Amsterdam (NL); Leigh, Mathew, Louis, Steven, 4132 Muttenz (CH); Schaich, Michael, 4058 Basel (CH)
(74) Representative: Hasler, Erich

(57) **Abstract**

The invention describes a solvent-free composition and a method for solubilizing a poorly water soluble compound by low to medium intensity sonication without damaging preformed liposomes. The invention discloses in a first container a poorly water soluble compound, preferably in aqueous suspension with D50 diameter in the range between 0.1 µm and 500 µm, preferably between 2 µm and 100 µm and a separate second container comprising an aqueous dispersion consisting of preformed standardized liposome particles characterized by Z-average diameter diameter between 30 nm and 200 nm, preferably between 30 nm and 100 nm; polydispersity index between 0.05 and 0.30; optical transmission of > 80%.

## Description

This invention relates to a method of solubilizing poorly water soluble compounds. In particular, it describes a solvent-free method of loading poorly water soluble drugs in liposomes for bulk production in small or large containers.

### BACKGROUND

Solvents are commonly employed to dissolve poorly water soluble compounds for *in vivo* studies for example by intravenous injection. Solvents may pose tolerability issues which impact on successful development of the drug. Co-solvent mixtures, acidic or alkaline pH adjustments and lipid carriers such as liposomes, micro-emulsions, micelles and mixed micelles are employed to solubilize poorly soluble compounds. Compounds solubilized in lipid particles may show better bioavailability because the drug is unlikely to precipitate out after injection, thereby providing higher blood concentrations in target organs.

Historically, poorly soluble compounds are mostly solubilized in liposomes by adding aqueous medium to a dried thin film. This thin film is difficult to scale up as it involves evaporating the solvent from an organic solution of a poorly soluble compound and phospholipids. Ultrasonication has been used to reduce the particle size of the liposomes formed from such films using sufficient power and intensity so that the Z-average diameter of the loaded liposomes can be in the region of 100 nm depending on the energy input. This process, however, can damage both the drug and liposomes because of the high intensity ultrasonication employed. Water soluble compounds are either dissolved in aqueous medium and added to the dried phospholipid film or added to phospholipids before film formation. High intensity sonication is applied to reduce liposome size which already contains a poorly soluble drug dissolved in the liposomes rather than suspended as solid particles in the aqueous medium.

There is an unmet need for a solvent-free parenteral solubilizer for poorly soluble drugs.

### PRIOR ART

EP-B-0 158 441 relates to pro-liposome compositions based on membrane lipids, to a method of making lipid vesicles by the addition of aqueous medium to the compositions and to aqueous dispersions of vesicles. The compositions may contain water or oil soluble biologically active compounds which are encapsulated in the aqueous phase of the liposomes or associated with the lipids during spontaneous *formation* of liposomes. It involves a volatile organic solvent.

WO97/25977 discloses a process for preparing an oil-in-water fat emulsion composition containing a cyclosporin, a rapamycin or an ascomycin or a derivative thereof, by mixing a placebo fat emulsion with a concentrate comprising the active compound, a stabiliser (e.g. phospholipid) and an organic solvent.

US-A-5,192,549 discloses a method of amphipathic drug loading into liposomes by applying a permanent pH gradient, whilst US-A-5,316,771 describes amphipathic drug loading into the aqueous compartment of the liposomes by ammonium ion gradient. US-A-5,380,531 also describes a method for an accumulation of amino acids and peptides into liposomes. The examples are confined to the loading of the aqueous compartment of the liposomes with hydrophilic very water soluble compounds at pH 7.2 (i.e. the compound is not in dispersion) possessing a basic function which can be ionised.

US-A-5,616,341, describes high drug lipid ratios in formulations comprising liposomal antineoplastic agents. The liposomes may be made by a process that loads the drug by an active mechanism using a trans membrane ion gradient, preferably a trans membrane pH gradient between the inside and outside of the lipid bilayers. Trapping efficiencies in the aqueous compartments of the liposomes are claimed to approach 100%. However, the method disclosed is restricted to loading water soluble (at pH 7.2) and ionizable antineoplastic agents in the internal aqueous domain of the vesicles. The undissocated form of the drug permeates the membrane and because of the pH gradient created, the more soluble salt form preferentially enriches the aqueous compartments, rather than associate with the lipid layers of the liposomes.

EP-B-0 560 138 describes the association of lipophilic drugs with liposomes by comixing the lipids and lipophilic drugs and other excipients, e.g. pH stabilisers, relying on high shear homogenisation for entrapment in lipid.

US 6,838,090 describes dosage forms wherein a compound with low water solubility is dissolved in organic solvents and added to liposome dispersions in a separate container.

WO 02/080883 describes a method of associating poorly soluble compounds in liposome dispersions. The method describes a two container system wherein a solution of the drug substance in hydrophilic solvents or a lyophilised amorphous powder of the drug substance prepared from a solution thereof in a first container is mixed with the contents in a second container containing a clear liposomal dispersion. The drug substance is dissolved in a solvent prior to loading in the lipid particles.

WO0156548 describes a liposome forming process in which an aqueous suspension containing empty liposomes formed of liposome forming compounds, a lipophilic active ingredient and dissolved sugar is dehydrated to solubilize the compound in liposomes.

In US-A-5653998, paclitaxel and other water insoluble compounds are formed into liposomal compositions which contain short chain fatty acids as stabilisers against flocculation. The formulations are formed by dispersing lipid and taxol directly into water using high energy mixing procedures, followed by filtration and high pressure homogenization.

The referenced disclosures for loading poorly water soluble drugs in liposomes require either (a) organic solvent for solubilizing poorly water soluble compounds in lipid particles (b) salt forms and pH adjustment for transfer to lipid domains (c) freeze-thaw cycles and/or high pressure homogenization to solubilize drugs and/or form liposomes at the same time (d) dehydrating and rehydrating dried films composed of phospholipid and drug in a mixture.

The prior art has failed to recognize that poorly soluble compounds can be solubilized in preformed liposomes without using solvents and without adding or solubilizing the drug in phospholipids to form a thin film before forming liposomes.

In the specification, the following definitions shall apply:
"liposomes" are vesicular structures including but not limited to small unilamellar vesicles (SUV) consisting of at least one neutral phospholipid including but not limited to, for example phosphytidylcholine (PC) and between 0.1% and 10% by weight of at least one charged phospholipid including but not limited to, for example phosphatidylglycerol (PG).

"low water solubility" or "poorly soluble" defines a compound that is lipophilic, hydrophobic or amphipathic and generally requires more than 1000 parts of water to dissolve 1 part of the compound at a pH between pH 6 and pH 8. The term in this specification concerns more specifically *very slightly soluble compounds* requiring from 1'000 to 10'000 parts of water or aqueous medium to dissolve 1 part of the compound. In particular the definition relates to *'practically insoluble or insoluble compounds'* requiring more than 10'000 parts of water to dissolve 1 part of the compound.

"solubilizing" in the context of the present method means generally dissolving for example physically associating at least 50%, preferably at least 80% more preferably at least 90% by weight of a poorly soluble compound in preformed and standardized liposomes with Z-average diameter between 30 nm and 200nm, preferably in clear liposome dispersions between 30 nm and 100nm with polydispersity index preferably between 0.05 and 0.3. The method of solubilizing is described as "solvent-free" or without using organic solvents to dissolve a poorly water soluble compound for loading in preformed liposomes.

"analytical filtration" refers to direct filtration of a solubilized liposome dispersion typically without dilution using a 220nm, preferably 100nm pore size filter wherein at least 50%, preferably at least 80 %, more preferably at least 90% by weight of the poorly soluble compound associates molecularly with the liposomes in the filtrate and less than 10% by weight of the solid drug in the aqueous medium is retained on the filter.

"preformed liposome dispersions" consist of "standardized liposomes" dispersed in aqueous medium characterised by z average particle diameter below 200nm preferably between 30 nm and 100 nm; preferably they are clear liposome dispersions further characterized by polydispersity index between 0.05 and 0.3; optical transmission of at least 80%, preferably at least 90% wherein Z-average diameter and polydispersity index of the standardized liposome dispersions are carried out by photon correlation spectrometry (PCS).

As used herein, the singular and plural terms are used interchangeably.

### OBJECT OF THE INVENTION

An object of the present invention is to provide a reproducible and up-scalable solvent-free method of solubilizing poorly soluble compounds in liposomes. The dispersion consisting of liposomes and solubilized compound is particularly useful for parenteral intravenous administration. The method is adaptable for solubilizing small amounts of available drugs in development and for bulk production in reaction vessels or in line.

### SUMMARY

According to the invention the objective is achieved by a system providing,
a) a first composition consisting of solid *particles* of a poorly water soluble compound with D50 diameter or Z-average diameter broadly in the range between 0.1 µm and 500 µm or a powder suspended in aqueous medium
b) a second aqueous dispersion consisting of preformed and standardized liposomes characterized by Z-average diameter below 200 nm, preferably between 30 nm and 100 nm; preferably clear liposome dispersions with polydispersity index between 0.05 and 0.30; optical transmission of>80%.

The invention further describes a solvent-free method of solubilization comprising,
a) combining said first composition consisting of solid particles with mean particle size in the broad range between 0.1 µm and 500 µm and said second composition consisting of preformed standardized liposomes
b) applying sufficient ultrasonic intensity to the dispersion and associating said poorly soluble compound with the said preformed liposomes until at least
   (i) > 90% by weight of the poorly soluble compound is solubilized by the preformed liposomes,
   (ii) Z-average diameter and polydispersity index of the loaded particles are within ± 20% of the values compared to the unloaded preformed liposome dispersion.

Particle size measurements are made using PCS after analytical filtration and can be confirmed by electron microscopy for example *cryo* Transmission Electron Microscopy (TEM).

The powder suspension in the first container and the preformed liposomes in the second container or reaction vessel may be kept apart in separate containers until the contents are sonicated using low to medium intensity ultrasonication without substantial damage to the preformed liposomes.

The method surprisingly solubilizes solid poorly water soluble compounds by ultrasonic cavitation and molecular diffusion at low to medium intensities without using solvents for dissolving the compound and without substantially changing the z average particle size and distribution characteristics of the preformed empty liposomes due to sonication. This is confirmed by the fact that the Z-average diameter of the liposome dispersion after sonication and drug loading are within ±20% of the values of the preformed dispersion before sonication and drug loading.

In one aspect the method may be used *in situ* for solubilizing small amounts of discovery compound or preparing explorative clinical service forms. Ultrasonication without causing substantial changes to the preformed liposomes is scalable for bulk production in batchwise reaction vessels or continuous inline processes containing preformed and standardized liposome dispersions. Typical energy/volume levels for such sonication and loading process without destruction of the preformed liposomes may be for example between 50Ws/mL to 1000 Ws/mL depending on processed volume and sonication time.

The fully loaded liposome dispersion which forms another aspect of the invention may be aseptically filtered and used for in vivo bioavailability studies by parenteral injection or oral gavage.

Non sterile compounds may be used because after loading, the resulting dispersion can be sterilised by aseptic filtration.

### DETAILED DESCRIPTION

The invention describes a solvent-free composition and a method for solubilizing a poorly water soluble compound by low to medium intensity sonication without damaging preformed liposomes. The invention discloses in a first container a poorly water soluble compound, preferably in aqueous suspension with D50 diameter or Z-average diameter in the range between 0.1 µm and 500 µm, preferably between 2 µm and 100 µm and a separate second container comprising an aqueous dispersion consisting of preformed standardized liposome particles characterized by Z-average diameter diameter between 30 nm and 200 nm, preferably between 30 nm and 100 nm; polydispersity index between 0.05 and 0.30; optical transmission of > 80%.

In one embodiment at least 50%, preferably at least 80%, more preferably at least 90% by weight of the poorly water soluble compound is solubilized in liposomes after a maximum of 60 minutes at room temperature (RT) by preferably low to medium intensity sonication for example between 0.2 µm and 60 µm amplitude at a frequency between 20kHz and 100kHz for solubilization in preformed liposome dispersions without substantial changes in the particle size of the preformed lipid dispersion for example within ± 20% of the z average particle diameter between 30 nm and 200 nm before loading.

In one embodiment the ultrasonication intensity is limited in that no metal particles are shed from the sonotrode in the liposome dispersion after ultrasonication, at least 50%, preferably at least 90% by weight of the poorly soluble drug is solubilized in liposomes, and the Z-average diameter of the liposomes are within ± 20% before ultrasonication and solubilization in preformed liposomes.

The embodiments include a separate second container consisting of preformed liposomes characterized by Z-average diameter between 30 nm and 200 nm, preferably between 30 nm and 100 nm; preferably in clear dispersions with polydispersity index between 0.05 and 0.30; optical transmission of >80%.

For compounds that are heat stable, for processing larger amounts for example > 500mL, the aqueous medium may be heated up to 100 °C, preferably up to 60 °C using higher intensity ultrasonication for example between 2 µm 20 µm amplitude, frequency between 20 kHz and 100 kHz and employing processing times for example below 15 minutes, limited in that the Z-average diameter of the liposomes are not substantially changed before and after sonication and solubilization in preformed liposomes.

In one embodiment the temperature of the sonication process is controlled at between 25°C and 60°C using for example flow-cells with temperature control.

In one embodiment for generating low to medium intensity sonication a bath sonicator with frequency between 20 kHz and 100 kHz and power output of between 100 watts and 200 watts may be used for containers holding between 1 mL and 500mL of preformed liposome diapersion.

Sufficient intensity ultrasonic energy may be generated in containers containing for example between 500 mL and 50 L of preformed liposome dispersion without substantial changes in the Z-average diameter of the liposomes therein by using probe sonicators for higher and more effective energy transfer and shorter sonication times for example below 30 minutes preferably below 15 mins

Compared to probe type sonicators, bath ultrasonicators are particularly suited for low intensity ultrasonication and *in situ* solubilizing using sterile containers containing preformed lipid dispersions, for example glass vials containing between 1mL and 10mL of preformed lipid dispersions for preclinical studies.

1000 watts probe sonicators may be used for immersion and direct contact with preformed lipid dispersions generating low intensity ultrasonication without substantially damaging liposomes. The ultrasonic device is suitable for solubilizing drugs in for example >500 mL liposome dispersion for development scale-up, bulk production in reaction vessels batch wise or streaming. Furthermore by the method of the invention, injectable liquid compositions may be aseptically prepared after sonication for parenteral (e.g. intravenous) use, or for oral dosage forms and therapies as well as topical applications which include pulmonary and occular dosage forms.

The loaded liposome dispersion containing the compound solubilized in preformed lipid particles may be sterilised by aseptic filtration or end sterilisation by e.g. autoclaving, depending on the stability of the compound. Furthermore, the loaded liposome dispersion may be frozen for storage and thawed using liposomes which are freezable. Alternatively, by removing water from the loaded lipid dispersion, solid compositions and dosage forms such as lyophilisates and tablets may be prepared.

In one embodiment the D50 diameter of the poorly water soluble compound between 0.1 µm and 500 µm is measured by laser diffraction.

In another embodiment the poorly soluble compound is a conventionally micronized powder, characterized by D50 diameter in the range between 1µ and 10µ, preferably between 2 µm and 5 µm and suspended in aqueous medium, optionally with 1% to 5% of surfactant for example soy or egg PC and/or polymers and/or hydrophilic liquid in the aqueous medium.

Micronized particles may be preferred because lower intensity ultrasonication and shorter sonication times can be adjusted to solubilize smaller particles in the aqueous medium into preformed liposomes without damaging the liposomes. Fragmentation of large particles requiring higher intensities which also damage liposomes is not necessary. Increased rate of solubilization in preformed liposomes is due to surface erosion of drug particles and increased diffusion from the aqueous medium.

### Biologically active compound

The final target ratio of the poorly soluble compound (in the first composition) to lipid (in the second composition) after the mixture is sonicated is typically within the range 1:1 and 1:1000, preferably 1:2 to 1:100, most preferably 1:5 to 1:50 parts by weight. Higher amounts of lipid increase drug load in the lipid domains. The powder or the suspension for loading by sonication may further include from 0.1% to 5% by weight, preferably 0.5% to 5% and most preferably between 0.5% and 3% of excipients such as surfactants and/or a hydrophilic liquid as wetting agent to facilitate wetting of the particles.

The compounds may be employed in e.g. CNS, oncology, cardiovascular diseases, metabolic, dermatology and antibiotic therapy, infectious diseases which include for example human immuno deficiency infection (HIV) and respiratory syncytial virus infection (RSV).

When the liposome dispersion is not sufficiently transparent, the loading process can nevertheless be followed by for example light microscopy to detect unassociated particles in the aqueous medium. Furthermore the fully loaded liposome dispersion is analytically filtered using 220 nm, preferably 100 nm pore size membrane filters to retain solid drug particles which are not solubilized in liposomes and remain on the filter. A feature of the invention is that water miscible organic solvents such as ethanol, DMSO and NMP are not employed to solubilize compounds that are poorly water soluble.

### Oral dosage forms

A solid composition which may be used for improving the solubility of poorly water soluble compound for oral use is prepared by removing water from the loaded liposome composition for example by spray drying. The composition may be processed with excipients into e.g. capsules, compacts or pellets. The process may be employed to solubilize suitable poorly water soluble compound in liposomes and improve the rate of dissolution thereby optimising bioavailability.

### Liposomes

The liposome dispersion comprises between 0.5% w/w to 25 % w/w, preferably below 20 % w/w, most preferably between 5 % w/w to 15 % w/w of at least one neutral and between 0.1% and 10% by weight of at least one negatively charged phospholipid in the lipid mixture.

The dispersions are preferably SUVs and preferably optically clear for example provides > 50%, preferably >90% light transmission for parenteral use.

Preformed liposomes may contain up to 10 % by weight, preferably 0.1 to 5 % of at least one charged phospholipid selected from the group of charged phospholipids consisting of phosphatidic acid (PA), phosphatitdyl inositol (PI), phosphatidylserine (PS) and phosphatidylglycerol (PG), to increase the capacity and surface area of the lipid domains in the lipid particles and allow for maximum association with lipophilic compounds. Preferably the lipid mixture comprising charged lipids, is homogeneously blended and dried from a substantially anhydrous solution. Solvent or solvent mixture is removed by lyophilization or spray drying to obtain the solid composition.

### Ultrasonic generator and processor

Ultrasonic device with appropriate power input and sonotrode can be adjusted to generate low to medium intensity ultrasonication for solubilizing solid particles using between 1 mL and 50L of preformed lipid dispersion without causing damage to preformed liposomes. In one embodiment for feasibility studies hand held ultrasonic device such as UP100H (Hielscher) at 100 watts power and 30kHz output and sonotrode MS 10 can be used for providing low intensity sonication for solubilization in volumes of up to 500mL.

Alternatively bath type sonicator such as Bandelin Sonorex Super RK 510 H (160 watts; 35kHz) can be used to solubilize 5/10mL in vials.

The lipid dispersion may be sterilised by aseptic filtration or preferably end sterilisation by autoclaving the loaded lipid dispersion in the final container. It is to be understood that the intensity and the total power required for sonication and drug loading without damaging the preformed liposomes or cause substantial change in the z average size of the preformed liposomes will depend on a number of factors. For example, the amount of liposome dispersion sonicated, concentration of phospholipid in the dispersion, viscosity, container size, temperature and sonication time.

### Phospholipid

Preferably the lipid dispersion contains at least one phospholipid of the formula 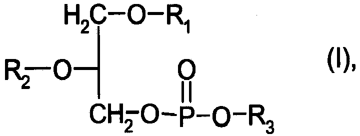 wherein,
R₁ represents C₁₀-C₂₀acyl;
R₂ represents hydrogen or C₁₀-C₂₀acyl;
R₃ represents hydrogen, 2-trimethylamino-1-ethyl, 2-amino-1-ethyl, C₁-C₄alkyl, C₁-C₅alkyl substituted by carboxy, C₂-C₅alkyl substituted by carboxy and hydroxy, C₂-C₅alkyl substituted by carboxy and amino, an inositol group or a glyceryl group or a salt of such compound.

Examples of neutral phospholipids with double chains are phosphatidylcholine (PC), phosphatidylethanolamine (PE).

Examples of charged phospholipids are phosphatidic acid (PA), phosphatitdyl inositol (PI) and phosphatidylserine (PS) and phosphatidylglycerol (PG). The hydrocarbon chain can either be unsaturated or saturated and can have between 10 and 24, preferably 14 and 18 carbon atoms.

The lipids may be derived from natural plant, or animal or microbiological sources, synthesised or partially synthesised. including polyethyleneglycol (PEG) derived phospholipid.

### Ultrasonic loading

Bandelin Sonorex Super RK 510 H (ultrasonicator & water bath in one):
- drug as a powder and 5 ml lipid dispersion in 6 ml type 1 glass vial on top of it
- no temperature control (water bath temperature after 60 minutes ultrasonication: 39.4°C)
- 60 minutes (5 minutes low intensity ultrasonication & 30 seconds shaking) for each formulation

Lipid dispersion before loading: z-average/pdI: 57.79 nm/0.104

### Example 1

Celecoxib at 5.5 mg/ml:

| After manufacturing: | Unfiltered | Filtered 0.22 (pvdf) |
|---|---|---|
| T[%]: | not determined | not determined |
| z-average/pdI: | 57.52 nm/0.103 | not determined |

| After T24-hours: | | |
|---|---|---|
| T[%]: | 88.93 | 90.90 |
| z-average/pdI: | 57.62 nm/0.084 | 57.62 nm/0.083 |

### Example 2

Cyclosporine at 6 mg/ml:

| After manufacturing: | Unfiltered. | Filtered 0.22 (pvdf) |
|---|---|---|
| T[%]: | not determined | not determined |
| z-average/pdI: | 55.81nm/0.160 | not determined |

| After T24-hours: | | |
|---|---|---|
| T[%]: | 88.93 | 90.90 |
| z-average/pdI: | 55.81nm/0.160 | 56.76 nm/0.160 |

It can be seen that the change in the physical characteristics of the preformed standardized lipid dispersion before and after loading and ultrasonication are not significantly changed and lie within ±20% of the empty preformed dispersion before loading.

Without limiting the scope of the invention preferred embodiments are described below.

In one embodiment a solvent-free method of solubilization comprises the steps of
a) forming a first composition consisting of solid particles of a poorly water soluble compound with D50 diameter in the broad range between 0.1 µm and 500 µm
b) providing a second composition consisting of preformed standardized liposomes characterized by Z-average diameter below 200 nm, preferably between 30 nm and 100 nm dispersed in preferably clear liposome dispersions with polydispersity index between 0.05 and 0.30; optical transmission of>80%
c) applying sufficient ultrasonic intensity to the dispersion and associating said poorl soluble compound with the said preformed liposomes until
   (i) at least 90% by weight of the poorly soluble compound is solubilized by the preformed liposomes preferably according to analytical filtration as defined and
   (ii) the z average particle size of the loaded liposomes is within ± 20% of the empty liposomes in the preformed liposome dispersion preferably determined by photon correlation spectroscopy and confirmed by cryo- electron microscopy.

In another embodiment the solubilized liposome dispersion is analytically filtered typically without dilution using a 220nm, preferably 100nm pore size filter wherein at least 50%, preferably at least 80 %, more preferably at least 90% by weight of the poorly soluble compound associates molecularly with the liposomes in the filtrate and less than 10% by weight of the solid drug in the aqueous medium is retained on the filter.

In a further embodiment for generating low to medium intensity ultrasonication a bath sonicator with frequency between 20 kHz and 100 kHz and power between 100 watts and 200 watts may be used for solubilizing solid particles in containers holding between 1 mL and 50 mL of preformed liposome dispersion.

In one embodiment for generating low to medium intensity ultrasonication an ultrasonic device can be used at oscillation amplitudes from about 1 µm to 60 µm and power between 100 watts and 1000 watts for solubilizing solid particles in containers holding between 50 mL and 50 L of preformed liposome dispersion.

In a further embodiment the combined first and second compositions are sonicated and maintained at a temperature range between 25 °C and 60 °C over a period of between 5 seconds and 60 minutes.

In yet another embodiment sufficient low to medium ultrasonic intensity is applied until at least 50%, preferably at least 95% by weight of the poorly soluble crystalline compound is associated in the preferably clear liposome dispersion without substantial damage or change in the z average particle diameter of the preformed liposomes.

In one embodiment the poorly water soluble compound is micronized and the mean particle diameter is in the range between 1µm and 10µm, preferably between 2 µm and 5 µm, optionally containing between 1% and 5% by weight of surfactants and/or polymers and/or hydrophilic liquid in the aqueous medium.

A particular embodiment further comprises a step of removing water for example by lyophilisation or spray drying from the loaded liposome dispersion for preparing solid compositions and dosage forms.

In one embodiment the liposome dispersion comprises between 0.5 % w/w to 25 % w/w, preferably below 20 % w/w, most preferably between 5 % w/w to 15 % w/w of at least one membrane lipid or preferably a phospholipid in the lipid mixture.

According to one embodiment the second composition may contain up to 10 % by weight, preferably 0.1 to 5 % of at least one charged phospholipid selected from the group of charged phospholipids consisting of phosphatidic acid (PA), phosphatitdyl inositol (PI), phosphatidylserine (PS) and phosphatidylglycerol (PG), optionally containing buffers, preferably, phosphate, citrate/phosphate, TRIS or histidine buffers.

According to another embodiment a solvent-free composition for solubilizing a poorly water soluble compound in a liposome dispersion comprising,
a) a first composition consisting of solid *particles* of a poorly water soluble compound with D50 diameter broadly in the range between 0.1 µm and 500 µm or a powder suspended in aqueous medium
b) a second aqueous dispersion consisting of preformed and standardized liposomes characterized by z average particle size below 200 nm, preferably between 30 nm and 100 nm; preferably clear liposome dispersions with polydispersity index between 0.05 and 0.30; optical transmission of>80%.

## Claims

1. A solvent-free method of solubilization comprising the steps of
a) forming a first composition consisting of solid particles of a poorly water soluble compound with D50 diameter in the broad range between 0.1 µm and 500 µm
b) providing a second composition consisting of preformed standardized liposomes **characterized by** Z-average diameter below 200 nm, preferably between 30 nm and 100 nm dispersed in preferably clear liposome dispersions with polydispersity index between 0.05 and 0.30; optical transmission of>80%
c) applying sufficient ultrasonic intensity to the dispersion and associating said poorly soluble compound with the said preformed liposomes until
(i) at least 90% by weight of the poorly soluble compound is solubilized by the preformed liposomes and
(ii) the Z-average diameter of the loaded liposomes is within ± 20% of the empty liposomes in the preformed liposome dispersion.

2. The method according to claim 1 **characterised in that**
- analytical filtration is employed in order to determine whether in step (i) said at least 90% by weight of the poorly soluble compound are solubilized by the preformed liposomes and/or
- photon correlation spectroscopy and confirmed by cryo- electron microscopy are employed in order to determine whether in step (ii) the Z-average diameter of the loaded liposomes is within ± 20% of the empty liposomes in the preformed liposome dispersion.

3. The method according to claim 1 or 2 wherein the solubilized liposome dispersion is analytically filtered typically without dilution using a 220nm, preferably 100nm pore size filter wherein at least 50%, preferably at least 80 %, more preferably at least 90% by weight of the poorly soluble compound associates molecularly with the liposomes in the filtrate and less than 10% by weight of the solid drug in the aqueous medium is retained on the filter.

4. The method according to any of claims 1 to 3 for generating low to medium intensity ultrasonication wherein a bath sonicator with frequency between 20 kHz and 100 kHz and power between 100 watts and 200 watts may be used for solubilizing solid particles in containers holding between 1 mL and 50 mL of preformed liposome dispersion.

5. The method according to any of claims 1 to 4 for generating low to medium intensity ultrasonication wherein an ultrasonic device can be used at oscillation amplitudes from about 1 µm to 60 µm and power between 100 watts and 1000 watts for solubilizing solid particles in containers holding between 50 mL and 50 L of preformed liposome dispersion.

6. The method according to any of claims 1 to 5 wherein the combined first and second compositions are sonicated and maintained at a temperature range between 25 °C and 60 °C over a period of between 5 seconds and 60 minutes.

7. The method according to any of claims 1 to 6 **characterised in that** sufficient low to medium ultrasonic intensity is applied until at least 50%, preferably at least 95% by weight of the poorly soluble crystalline compound is associated in the preferably clear liposome dispersion without substantial damage or change in the z average particle diameter of the preformed liposomes.

8. The method according to any of claims 1 to 8 **characterised in that** the poorly water soluble compound is micronized and the mean particle diameter is in the range between 1µm and 10µm, preferably between 2 µm and 5 µm, optionally containing between 1% and 5% by weight of surfactants and/or polymers and/or hydrophilic liquid in the aqueous medium.

9. The method according to any of claims 1 to 8 further comprising a step of removing water from the loaded liposome dispersion by lyophilisation or spray drying for preparing solid compositions and dosage forms.

10. The method according to any of claims 1 to 9 **characterised in that** the liposome dispersion comprises between 0.5 % w/w to 25 % w/w, preferably below 20 % w/w, most preferably between 5 % w/w to 15 % w/w of at least one membrane lipid or preferably a phospholipid in the lipid mixture.

11. The method according to any of claims 1 to 10 **characterised in that** the second composition may contain up to 10 % by weight, preferably 0.1 to 5 % of at least one charged phospholipid selected from the group of charged phospholipids consisting of phosphatidic acid (PA), phosphatitdyl inositol (PI), phosphatidylserine (PS) and phosphatidylglycerol (PG), optionally containing buffers, preferably, phosphate, citrate/phosphate, TRIS or histidine buffers.

12. A solvent-free composition for solubilizing a poorly water soluble compound in a liposome dispersion comprising,
a) a first composition consisting of solid *particles* of a poorly water soluble compound with D 50 diameter broadly in the range between 0.1 µm and 500 µm or a powder suspended in aqueous medium
b) a second aqueous dispersion consisting of preformed and standardized liposomes **characterized by** Z-average diameter below 200 nm, preferably between 30 nm and 100 nm; preferably clear liposome dispersions with polydispersity index between 0.05 and 0.30; optical transmission of >80%.
